# EUROPÄISCHE PATENTSCHRIFT

(11) **EP 1 601 391 B1**
(45) Veröffentlichungstag und Bekanntmachung des Hinweises auf die Patenterteilung: **05.07.2006**
(21) Anmeldenummer: 04713053.9
(22) Anmeldetag: 20.02.2004
(51) Int. Cl.: A61L 27/54, A61L 29/16, A61L 31/16, A61L 33/00

(54) **REDUZIERUNG DER KOMPLEMENT-AKTIVIERUNG BEI MEDIZINISCHEN VORRICHTUNGEN**
REDUCTION OF COMPLEMENT ACTIVATION IN MEDICAL DEVICES
REDUCTION DE L'ACTIVATION DU COMPLEMENT SUR DES DISPOSITIFS MEDICAUX

(30) Priorität: 20.02.2003 DE 10307205
(43) Veröffentlichungstag der Anmeldung: 07.12.2005
(73) Patentinhaber: Nakel, Mathias, 72393 Burladingen/Ringingen (DE)
(72) Erfinder: Nakel, Mathias, 72393 Burladingen/Ringingen (DE)
(74) Vertreter: Otten, Hajo
(86) Internationale Anmeldenummer: PCT/EP2004/001685
(87) Internationale Veröffentlichungsnummer: WO 2004/073762

(56) Entgegenhaltungen:
- WO-A-97/35886
- WO-A-98/34959
- US-A- 4 847 338
- US-A1- 2002 198 590
- US-B1- 6 200 588
- SOULIKA A M ET AL: "Inhibition of Heparin/Protamine Complex-Induced Complement AcTIVATION BY COMPstatin in Baboons" September 2000 (2000-09), CLINICAL IMMUNOLOGY, ACADEMIC PRESS,, US, PAGE(S) 212-221 , XP002244612 ISSN: 1521-6616 Seite 212 - Seite 213

## Beschreibung

Die vorliegende Erfindung betrifft die Reduzierung der Komplement-Aktivierung von Kunststoffoberflächen, die mit einer Beschichtung aus einem ionischen Komplex aus anionischem Heparin oder Heparin-Derivat und kationischem Tensid versehen sind, eine entsprechende Beschichtung für Kunststoffoberflächen sowie eine medizinische Vorrichtung mit zumindest einer Kunststoffoberfläche mit einer derartigen Beschichtung.

Derartige Verfahren, Beschichtungen und medizinische Vorrichtungen sind aus dem Stand der Technik vielfach bekannt. Bei vielen medizinischen Behandlungen werden Kunststoffoberflächen verwendet, die über längere oder kürzere Zeiträume mit dem Blut eines Patienten in Kontakt kommen. Bei derartigen Vorrichtungen handelt es sich beispielsweise um Einmalgeräte für eine Herz-Lungen-Maschine, einen Oxygenator, einen Katheter, ein künstliches Herz, eine künstliche Niere, eine Gasaustauschmembran oder eine vaskuläre Prothese, wobei diese Aufzählung keineswegs als abschließende Aufzählung zu verstehen ist.

Um die Koagulation des mit diesen Kunststoffoberflächen in Kontakt kommenden Blutes zu verhindern, ist es generell erforderlich, diese Kunststoffoberflächen mit antithrombotischen Eigenschaften zu versehen. Zu diesem Zweck wird auf die Kunststoffoberflächen häufig eine heparinhaltige Beschichtung aufgebracht.

Aus der US 6,200,588 B1 ist in diesem Zusammenhang ein Verfahren zur blutverträglichen Beschichtung von Kunststoffoberflächen bekannt.

Zur Herstellung der Beschichtung wird aus einer Lösung ein ionischer Komplex aus anionischem Heparin und kationischem Tensid als stabil haftende Beschichtung auf der Kunststoffoberfläche abgelagert. Das kationische Tensid ist dabei eine Ammoniumverbindung mit vier aliphatischen Alkylgruppen,

Versuche im Hause der Anmelderin haben nun überraschend gezeigt, dass eine derartige Beschichtung zwar antithrombogene Eigenschaften aufweist, im Gegensatz zu anderen bekannten Heparinbeschichtungen wie beispielsweise der Beschichtung "Bioline" der Anmelderin bei Kontakt mit Humanblut eine unverhältnismäßig hohe Komplement-Aktivierung erzeugt. Die bekannte Beschichtung entspricht damit in diesem einen Punkt nicht dem, was unter guter Blutverträglichkeit verstanden wird.

Gute Blutverträglichkeit bedeutet für eine Oberfläche nämlich, dass sie bei Kontakt mit Blut weder die Blutgerinnung noch die Abwehrmechanismen des Körpers gegen die Fremdoberfläche anstößt.

Gerade die bei der bekannten Beschichtung überraschenderweise gefundene hohe Komplement-Aktivierung ist besonders von Nachteil, denn sie führt zu systemischen Entzündungen und kann beispielsweise postoperatives Organversagen verursachen.

In der Literatur wird vor diesem Hintergrund eine Reduzierung der Komplement-Aktivierung umfassend diskutiert; siehe beispielsweise Videm et al. in J. Thorac. Cardiovasc. Surg. 1992; 103: 806-813, und Baufreton et al., Perfusion 1998; 13: 419-427.

Vor diesem Hintergrund liegt der vorliegenden Erfindung die Aufgabe zugrunde, das eingangs genannte Beschichtung derart weiterzubilden, dass die Komplement-Aktivierung verringert wird.

Erfindungsgemäß wird diese Aufgabe gelöst durch die Verwendung von Lecithin pflanzlichen Ursprungs als zwitterionisches Netzmittel zur Reduzierung der Komplement-Aktivierung einer mit einer Beschichtung aus einem ionischen Komplex aus anionischem Heparin oder Heparin-Derivat und kationischem Tensid versehenen Kunststoffoberfläche.

Die der Erfindung zugrunde liegende Aufgabe wird auf diese Weise vollkommen gelöst.

Der Erfinder der vorliegenden Anmeldung hat nämlich überraschenderweise festgestellt, dass die Verwendung von Lecithin pflanzlichen Ursprungs als zwitterionisches Netzmittel in der an sich bekannten Beschichtung dazu führt, dass die Komplement-Aktivierung gegenüber der bekannten Beschichtung allein verringert wird. Die Erfinder konnten ferner feststellen, dass es möglich ist, geeignete weitere Substanzen auszuwählen, die die Komplement-Aktivierung verringern, nicht jedoch zu einer Verringerung der Stabilität der Beschichtung führen. Mit anderen Worten, durch die nun erfindungsgemäß zusätzlich in der Beschichtung vorgesehene weitere Substanz bleibt die Haftung der Beschichtung auf der Kunststoffoberfläche erhalten, es ist kein oder zumindest kein wesentlicher Verlust der Stabilität zu verzeichnen. Dies bedeutet, dass auch bei der neuen Beschichtung beispielsweise bei einem Langzeitkontakt der mit einer erfindungsgemäßen Beschichtung versehenen Kunststoffoberfläche mit Blut das Heparin nicht herausgewaschen wird und somit die antithrombogenen Eigenschaften erhalten bleiben.

In einer Weiterbildung wird die Beschichtung aus einer Lösung oder wässrigen Emulsion, die den anionischen Komplex und das zwitterionische Netzmittel enthält, als haftende Beschichtung stabil auf der Kunststoffoberfläche abgelagert.

Bei dieser Maßnahme ist von Vorteil, dass sie technisch einfach durchzüführen ist. Das bekannte Beschichtungsverfahren kann in der üblichen Weise durchgeführt werden, wobei es lediglich erforderlich ist, zusätzlich zu dem ionischen Komplex noch das zwitterionische Netzmittel Lecithin pflanzlichen Ursprungs in die Lösung oder die wässrige Emulsion zu geben. Damit können bekannte Prozessabläufe weitgehend beibehalten werden.

Der Erfinder der vorliegenden Anmeldung konnte feststellen, dass bei Verwendung von Lecithin pflanzlichen Ursprungs als zwitterionisches Netzmittel einerseits die Benetzbarkeit der Beschichtung deutlich verbessert wird, andererseits aber verhindert wird, dass der ionische Komplex wieder aus der Beschichtung herausgewaschen wird, wie es beispielsweise der Fall wäre, wenn schärfere Tenside verwendet würden.

Dabei ist es besonders bevorzugt, wenn Lecithin pflanzlichen Ursprungs als Netzmittel Soja-Lecithin ist.

Bei dieser Maßnahme ist von Vorteil, dass als weitere Substanz eine Zusammensetzung verwendet wird, die prinzipiell bei mit Blut in Kontakt kommenden Oberflächen seit längerer zeit eingesetzt wird und deren Unbedenklichkeit in diesem Zusammenhang bekannt ist. In diesem Zusammenhang wird auf die US 6,482,630 B2 verwiesen, die die Verwendung von Lecithin in einem Antischäumungsmittel zeigt.

Die Verwendung von Lecithin pflanzlichen Ursprungs, vorzugsweise von Soja-Lecithin, hat den Vorteil, dass verhindert wird, dass auch nur minimale Restverunreinigungen aus tierischen Organismen in die Beschichtung eingelagert werden können, was insbesondere aus Gründen der Immunverträglichkeit von Vorteil ist.

Aus dem Stand der Technik sind zwar Oberflächenbeschichtungen bekannt, die Heparin und vernetzte Lecithinderivate enthalten, um die antithrombogenen Eigenschaften zu verbessern, dass der-artige Oberflächenbeschichtungen jedoch die Komplement-Aktivierung reduzieren, war bisher nicht bekannt.

So beschreibt der Artikel von De Somer et al.: Phosphorylcholine coating offers natural platelet preservation during cardiopulmonary bypass, Perfusion 2002, 17 : 39 - 44 unter anderem auch eine Untersuchung zur Komplement-Aktivierung durch Lecithin, wobei jedoch kein Unterschied zwischen beschichteten und unbeschichteten Oberflächen festgestellt wurde.

Die z.B. aus der US 5,496,581, US 5,422,402 oder US 5,418,295 bekannten Verfahren, die vernetzte Lecithinderivate verwenden, sind zudem technisch sehr aufwändig.

Der Erfinder der vorliegenden Anmeldung hat nun erkannt, dass durch die oben beschriebenen, einfachen Verfahrensschritte, bei denen Lecithin pflanzlichen Ursprungs zugegeben wird, entgegen der in dem o.g. Artikel von De Somer gezeigten Ergebnisse doch eine Reduzierung der Komplement-Aktivierung zu verzeichnen ist.

Allgemein ist es bevorzugt, wenn die weitere Substanz in der Lösung oder Emulsion zu weniger als 1 Gew.-%, vorzugsweise zu weniger als 0,1 Gew.-%, weiter vorzugsweise zu ca. 0,05 Gew.-% enthalten ist.

Der Erfinder der vorliegenden Anmeldung hat erkannt, dass bereits diese geringen Anteile der weiteren Substanz in der Lösung zu einer deutlichen Verbesserung der Benetzbarkeit der Beschichtung führen.

Weiter ist es bevorzugt, wenn in der Lösung oder wässrigen Emulsion als Additiv zumindest ein nicht-ionischer Weichmacher, insbesondere Cyclohexan-1,2-dicarbonsäure-bis-2-ethylhexylester (DINCK) oder 1,2,4-Benzol-tricarbonsäure-tris-2-ethylhexylester oder ein Öl, insbesondere Rizinusöl (Castor Oil) oder Sojaöl, vorhanden ist.

Bei dieser Maßnahme ist von Vorteil, dass die Komplement-Aktivierung noch weiter reduziert wird, sie liegt nach ersten Messungen des Erfinders sogar noch unter dem Wert für unbeschichtete Kunststoffoberflächen.

Allgemein ist es bevorzugt, wenn das kationische Tensid im Heparinkomplex eine Ammoniumverbindung mit vier aliphatischen Alkylgruppen ist, wobei die Ammoniumverbindung vorzugsweise ein Di-methyl-di-alkyl(C10 bis C18)-Ammonium-Kation ist.

Eine derartige Zusammensetzung des kationischen Tensides ist aus der eingangs erwähnten US 6,200,588 B1 bekannt.

Weiter ist es bevorzugt, wenn der ionische Komplex zu weniger als 1 Gew.-%, vorzugsweise zu ca. 0,1 bis 0,4 Gew.-%, in der Lösung oder Emulsion enthalten ist, wobei die Lösung vorzugsweise ein organisches Lösungsmittel enthält.

Der Erfinder der vorliegenden Anmeldung hat erkannt, dass diese Merkmale zusammen mit Lecithin pflanzlichen Ursprungs als zwitterionisches Netzmittel zu einer besonders gut benetzbaren und stabil auf der Kunststoffoberfläche haftenden Beschichtung führen, bei der die Komplement-Aktivierung reduziert ist.

Dabei ist es weiter bevorzugt, wenn das Lösungsmittel polaren Alkohol und unpolaren Kohlenwasserstoff, vorzugsweise im Verhältnis von etwa 1:5 enthält.

Diese Maßnahme ist unter verfahrenstechnischen Gesichtspunkten von Vorteil, denn in einer derartigen Lösung lassen sich der ionische Komplex sowie die weitere Substanz gut in Lösung bringen, wobei nach dem Abdunsten des Lösungsmittels ein fest auf der Oberfläche haftender Film entsteht.

Vor diesem Hintergrund betrifft die vorliegende Erfindung ferner eine Beschichtung für Kunststoffoberflächen mit reduzierter Komplement-Aktivierung, in der ein zwitterionisches Netzmittel erfindungsgemäß verwendet wird.

Schließlich betrifft die vorliegende Erfindung auch eine medizinische Vorrichtung mit zumindest einer Kunststoffoberfläche, die eine erfindungsgemäße Beschichtung aufweist.

Die medizinische Vorrichtung ist dabei vorzugsweise Bestandteil einer mit Blut in Kontakt kommenden Vorrichtung, wie beispielsweise von Einmalgeräten einer Herz-Lungen-Maschine, eines Oxygenators, eines Katheters, eines künstlichen Herzens, einer künstlichen Niere, einer Gasaustauschmembran oder einer vaskulären Prothese.

Weiter vorzugsweise besteht die Kunststoffoberfläche aus Polypropylen (PP), Polycarbonat (PC), Poly-4-methyl-pent-1-en (PMP), Polyurethan (PU), Polyethylen (PE), Polyester, Silikon, Hart- oder Weich-Polyvinylchlorid (PVC).

Weitere Vorteile ergeben sich aus der beigefügten Beschreibung.

### Beispiel 1: Herstellen des ionischen Komplexes

Wie im Beispiel 3 der eingangs erwähnten US 6,200,588 B1 beschrieben, wird ein ionischer Komplex aus Heparin und einer Ammoniumverbindung mit vier aliphatischen Alkylgruppen wie folgt hergestellt:

Di-methyl-di-dodecyl-ammoniumchlorid (6 Teile) und Di-methyl-di-tetradecyl-ammoniumchlorid (19 Teile) werden zu Methanol (25 Teile) unter Rühren hinzugegeben und gelöst. Danach wird Wasser bis zu einer Konzentration von 70 % hinzugegeben, wobei ein Teil des Ammoniumsalzes ausfällt.

Die entstandene Suspension wird auf 50°C erhitzt, um eine homogene Lösung zu erzeugen. Dann wird Heparin (10 Teile) in Wasser (25 Teile) gelöst und Methanol wird bis zu einer Konzentration von 30 % hinzugegeben. Bei diesem Schritt fällt ebenfalls ein Teil des Heparins aus, so dass eine Suspension entsteht.

Diese Suspension wird auf 70°C erhitzt, um eine homogene Lösung herzustellen.

Die Lösung des Ammoniumsalzes wird tropfenweise zu der Heparinlösung unter Rühren hinzugegeben. Das Reaktionsprodukt ist in dem Lösungsmittel unlöslich und fällt sofort aus. Das Präzipitat wird abfiltriert und gründlich gewaschen, um freies Heparin und Ammoniumsalz zu entfernen.

Schließlich wird das Präzipitat zentrifugiert, um Wasser zu entfernen, und abschließend lyophilisiert, was ein weißes Puder ergibt.

### Beispiel 2: Herstellen einer beschichteten Kunststoffoberfläche

Das weiße Pulver aus Beispiel 1 wird in einem Lösungsmittel aus 20 Gew.-% Propanol und 80 Gew.-% Heptan gelöst. In diese Lösung wird zusätzlich Soja-Lecithin gegeben und unter Rühren ebenfalls aufgelöst.

Die Endkonzentrationen sind 99,75 Gew.-% Lösungsmittel, 0,2 Gew.-% ionischer Komplex aus Beispiel 1 und 0,05 Gew.-% Soja-Lecithin.

Diese Lösung wird auf eine hydrophobe Kunststoffoberfläche aus Polycarbonat aufgebracht. Nach dem Abdunsten des Lösungsmittels verbleibt ein fest auf der Oberfläche haftender Film, der gegenüber einer Beschichtung ohne Lecithin-Anteil eine verbesserte Benetzbarkeit aufweist.

In die Lösung kann zur weiteren Reduzierung der Komplement-Aktivierung noch ein Additiv gegeben werden, wobei dann 0,15 Gew.-% ionischer Komplex aus Beispiel 1, 0,05 Gew.-% Soja-Lecithin und 0,075 Gew.-% Additiv als Endkonzentration in der Lösung enthalten sind. Die Endkonzentration des Lösungsmittels beträgt dann entsprechend 99,725 Gew.-%.

Folgende Additive wurden bisher getestet:
- A:: Rizinusöl (Castor Oil),
von Caesar & Lorentz GmbH, Hilden, Deutschland
- B:: Sojaöl
von Caesar & Lorentz GmbH, Hilden, Deutschland
- C:: Cyclohexan-1,2-dicarbonsäure-bis-2-ethylhexylester
von BASF, Ludwigshafen, Deutschland
- D:: 1,2,4-Benzol-tricarbonsäure-tris-2-ethylhexylester
von Acros Organics, Geel, Belgien.

Alternativ zur direkten Beschichtung aus organischem Lösungsmittel kann die Beschichtung auch aus einer wässrigen Emulsion ausgeführt werden, wenn die zu beschichtetende Oberfläche Materialien enthält, die von organischen Lösungsmitteln entweder angegriffen werden (z.B. Polycarbonat) oder gespalten werden (z.B. Silikon).

Die wie oben hergestellten Lösungen werden hierfür in Wasser emulgiert und das Lösungsmittel entfernt. Das Herstellen wässriger Emulsionen aus Lösungen ist im Stand der Technik hinreichend bekannt.

### Beispiel 3: Bestimmung der Komplement-Aktivierung

In einem Chandler-Loop-Test, wie er beispielsweise beschrieben ist in Wendel et al., Journal of Biomaterials Application, 17:5-, wird die Hämokompatibilität der im Beispiel 2 beschichteten Kunststoffoberfläche mit frischem Humanblut getestet.

Zu diesem Zweck werden sieben Konnektoren aus Polycarbonat so mit der neuen Beschichtung versehen, wie dies in Beispiel 2 beschrieben ist. Zum Vergleich werden sieben weitere Konnektoren aus Polycarbonat mit einer Beschichtung versehen, wie dies in Beispiel 2 beschrieben ist, wobei jedoch das Lecithin weggelassen wurde. Die Vergleichsbeschichtung entspricht damit der aus der eingangs erwähnten US 6,200,588 B1 beschriebenen Beschichtung.

Die sieben Konnektoren aus Polycarbonat wurden jeweils mit 3/8'' Silikon-Schlauch zu einem insgesamt 50 cm langen geschlossenen Ring verbunden. Dieser Ring wies auf seiner Innenseite 90 % PC-Oberfläche und 10 % Silikon-Oberfläche auf.

Der Ring wird jeweils mit 20 ml frischem Humanblut mit 1 IU/ml Heparin gefüllt und bei 37°C für 90 Minuten mit 30 Umdrehungen pro Minute geschleudert.

Anschließend werden verschiedene Blutparameter ermittelt, wie dies in der oben erwähnten Veröffentlichung von Wendel et al. beschrieben ist.

Zur Bestimmung der Komplement-Aktivierung wurde der TCC (SC5b-9), also der terminal complement complex mit einem ELISA-Test ermittelt, der von Quidel, San Diego, CA, USA, bezogen wurde.

Neben der erfindungsgemäßen sowie der bekannten Beschichtung (US 6,200,588 B1) wurden auch unbeschichtete Konnektoren aus Polycarbonat in dem oben beschriebenen Chandler-Loop-Test untersucht. Jeder der drei Ringe wurde dabei sechsmal mit frischem Humanblut vermessen und aus den jeweils sechs Messwerten ein Durchschnittswert ermittelt.

Für die Komplement-Aktivierung ergaben sich in einer ersten Messreihe dabei folgende Werte:

| | |
|---|---|
| Unbeschichtet: | 1094 |
| Bekannte Beschichtung: | 3423 |
| Neue Beschichtung: | 1603 |

In einer zweiten Messreihe mit Blut von einem anderen Spender ergaben sich folgende Werte:

| | |
|---|---|
| Unbeschichtet: | 1090 |
| Bekannte Beschichtung: | 2292 |
| Neue Beschichtung ohne Additiv: | 930 |
| Neue Beschichtung mit Additiv A: | 510 |
| Neue Beschichtung mit Additiv B: | 644 |
| Neue Beschichtung mit Additiv C: | 841 |
| Neue Beschichtung mit Additiv D: | 778 |

Es ist zu erkennen, dass durch die neue Beschichtung die Komplement-Aktivierung verglichen mit der aus der US 6,200,588 B1 bekannten Beschichtung mehr als halbiert wurde.

Durch die Hinzugabe eines der Additive A bis D wird die Komplement-Aktivierung sogar unter die Komplement-Aktivierung der unbeschichteten Chandler-Loops reduziert.

Bei den anderen, im Chandler-Loop-Test ermittelten Blutparametern ergab sich kein signifikanter Unterschied zwischen der bekannten und der neuen Beschichtung, wohl aber eine deutliche Verbesserung gegenüber der unbeschichteten Kontrolle. Dies gilt insbesondere für die Platelet-Zahl, β-Thromboglobulin, TAT (Thrombin-Anti-Thrombin-Komplex) und PMN-Elastase (aus aktivierten polymononuklearen Leukozyten).

Aus den vergleichenden Messergebnissen der oben beschriebenen Chandler-Loop-Tests ergibt sich, dass die Komplement-Aktivierung durch die neue Beschichtung deutlich verringert wurde, während die sonstigen Blutparameter nach wie vor gut sind, was darauf hindeutet, dass die neue Beschichtung stabil auf der Kunststoffoberfläche haftet.

## Patentansprüche

1. Verwendung eines zwitterionischen Netzmittels zur Reduzierung der Komplement-Aktivierung einer Kunststoffoberfläche, welche mit einer Beschichtung aus einem ionischen Komplex aus anionischem Heparin oder Heparin-Derivat und kationischem Tensid versehen ist, wobei das zwitterionische Netzmittel Lecithin pflanzlichen Ursprungs ist.

2. Verwendung nach Anspruch 1, **dadurch gekennzeichnet, dass** das Lecithin Soja-Lecithin ist.

3. Verwendung nach Anspruch 1 oder 2, **dadurch gekennzeichnet, dass** die Beschichtung aus einer Lösung oder wässrigen Emulsion besteht, die den ionischen Komplex und das zwitterionische Netzmittel enthält, und dass in der Lösung oder wässrigen Emulsion zumindest ein nicht-ionischer Weichmacher, insbesondere Cyclohexan-1,2-dicarbonsäure-bis-2-ethylhexylester oder 1,2,4-benzol-tricarbonsäure-tris-2-6-ethylhexylester oder ein Öl, insbesondere Rizinusöl oder Sojaöl, vorhanden ist.

4. Verwendung nach Anspruch 3, **dadurch gekennzeichnet, dass** das zwitterionische Netzmittel in der Lösung oder wässrigen Emulsion zu weniger als 1 Gew.-%, vorzugsweise zu weniger als 0,1 Gew.-% enthalten ist.

5. Verwendung nach Anspruch 4, **dadurch gekennzeichnet, dass** das zwitterionische Netzmittel in der Lösung oder wässrigen Emulsion zu ca. 0,05 Gew.-% enthalten ist.

6. Verwendung nach einem der Ansprüche 2 bis 5, **dadurch gekennzeichnet, dass** der nicht-ionische Weichmacher in der Lösung oder wässrigen Emulsion zu weniger als 1 Gew.-%, vorzugsweise zu weniger als 0,1 Gew.-% enthalten ist.

7. Verwendung nach Anspruch 6, **dadurch gekennzeichnet, dass** der nicht-ionische Weichmacher in der Lösung oder wässrigen Emulsion zu ca. 0,075 Gew.-% enthalten ist.

8. Verwendung nach einem der Ansprüche 1 bis 7, **dadurch gekennzeichnet, dass** das kationische Tensid im Heparinkomplex eine Ammoniumverbindung mit vier aliphatischen Alkylgruppen ist.

9. Verwendung nach Anspruch 8, **dadurch gekennzeichnet, dass** die Ammoniumverbindung ein Di-methyl-di-alkyl(C10 bis C18)-Ammonium-Kation ist.

10. Verwendung nach Anspruch 8 oder 9, **dadurch gekennzeichnet, dass** der ionische Komplex zu weniger als 1 Gewichtsprozent, vorzugsweise zu ca. 0,1 bis 0,4 Gewichtsprozent in der Lösung oder wässrigen Emulsion enthalten ist.

11. Verwendung nach einem der Ansprüche 2 bis 10, **dadurch gekennzeichnet, dass** die Lösung ein organisches Lösungsmittel enthält.

12. Verwendung nach Anspruch 11, **dadurch gekennzeichnet, dass** das organische Lösungsmittel polaren Alkohol und unpolaren Kohlenwasserstoff, vorzugsweise im Verhältnis von etwa 1:5 enthält.

13. Beschichtung für Künststoffaberflächen mit reduzierter Komplement-Aktivierung aus einem ionischen Komplex aus anionischem Heparin oder Heparin-Derivat und kationischem Tensid , in der ein zwitterionisches Netzmittel wie in einem der Ansprüche 1 bis 12 verwendet wird.

14. Medizinische Vorrichtung mit zumindest einer Kunststoffoberfläche, die eine Beschichtung nach Anspruch 13 aufweist.

15. Medizinische Vorrichtung nach Anspruch 14, **dadurch gekennzeichnet, dass** sie Bestandteil einer mit Blut in Kontakt kommenden Vorrichtung, vorzugsweise von Einmalgeräten einer Herz-Lungen-Maschine, eines oxygenators, eines Kathethers, eines künstlichen Herzens, einer künstlichen Niere, einer Gasaustauschmembran oder einer vaskulären Prothese ist.

16. Medizinische Vorrichtung nach Anspruch 14 oder 15, **dadurch gekennzeichnet, dass** die Kunststoffoberfläche aus PP, PC, PMP, PU, PE, Polyester, Silikon, Hart- oder Weich-PVC besteht.

## Claims

1. use of a zwitterionic wetting agent for reducing complement activation of a plastics surface which is provided with a coating of an ionic complex of anionic heparin or heparin derivative and cationic surfactant, where the zwitterionic wetting agent is lecithin of plant origin.

2. The use as claimed in claim 1, **characterized in that** the lecithin is soybean lecithin.

3. The use as claimed in claim 1 or 2, **characterized in that** the coating consists of a solution or aqueous emulsion which comprises the ionic complex and the zwitterionic wetting agent, and **in that** at least one nonionic plasticizer, in particular cyclohexane-1,2-dicarboxylic acid bis-2-ethylhexyl ester or 1,2,4-benzenetricarboxylic acid tris-2-ethylhexyl ester or an oil, in particular castor oil or soybean oil, is present in the solution or aqueous emulsion.

4. The use as claimed in claim 3, **characterized in that** the content of zwitterionic wetting agent in the solution or aqueous emulsion is less than 1% by weight, preferably less than 0.1% by weight.

5. The use as claimed in claim 4, **characterized in that** the content of zwitterionic wetting agent in the solution or aqueous emulsion is about 0.05% by weight.

6. The use as claimed in any of claims 2 to 5, **characterized in that** the content of nonionic plasticizer in the solution or aqueous emulsion is less than 1% by weight, preferably less than 0.1% by weight.

7. The use as claimed in claim 6, **characterized in that** the content of nonionic plasticizer in the solution or aqueous emulsion is about 0.075% by weight.

8. The use as claimed in any of claims 1 to 7, **characterized in that** the cationic surfactant in the heparin complex is an ammonium compound having four aliphatic alkyl groups.

9. The use as claimed in claim 8, **characterized in that** the ammonium compound is a dimethyldialkyl(C10 to C18)ammonium cation.

10. The use as claimed in claim 8 or 9, **characterized in that** the content of ionic complex in the solution or aqueous emulsion is less than 1 percent by weight, preferably about 0.1 to 0.4 percent by weight.

11. The use as claimed in any of claims 2 to 10, **characterized in that** the solution comprises an organic solvent.

12. The use as claimed in claim 11, **characterized in that** the organic solvent comprises polar alcohol and nonpolar hydrocarbon, preferably in the ratio of about 1:5.

13. A coating for plastics surfaces with reduced complement activation, with an ionic complex of anionic heparin or heparin derivative and cationic surfactant, in which a zwitterionic wetting agent is used as claimed in any of claims 1 to 12.

14. A medical device having at least one plastics surface which has a coating as claimed in claim 13.

15. The medical device as claimed in claim 14, **characterized in that** it is part of a device which comes into contact with blood, preferably of disposable appliances of a heart-lung machine, of an oxygenator, of a catheter, of an artificial heart, of an artificial kidney, of a gas exchange membrane or of a vascular prosthesis.

16. The medical device as claimed in claim 14 or 15, **characterized in that** the plastics surface consists of PP, PC, PMP, PU, PE, polyester, silicone, rigid or plasticized PVC.

## Revendications

1. Utilisation d'un agent mouillant zwitterionique pour réduire l'activation du complément d'une surface en matière plastique qui est munie d'un revêtement constitué par un complexe ionique d'héparine anionique ou d'un dérivé d'héparine anionique et d'un tensioactif cationique, où l'agent mouillant zwitterlonique est une lécithine d'origine végétale.

2. Utilisation selon la revendication 1, **caractérisée en ce que** la lécithine est de la lécithine de soja.

3. Utilisation selon la revendication ou 2, **caractérisée en ce que** le revêtement consiste en une solution ou émulsion aqueuse qui contient le complexe ionique et l'agent mouillant zwitterionique et **en ce que**, dans la solution ou l'émulsion aqueuse, au moins un plastifiant non ionique, en particulier le cyclohexane-1,2-dicarboxylate de bis-2-éthylhexyle ou le 1,2,4-benzène-tricarboxylate de tris-2,6-éthylhexyle ou une huile, en particulier l'huile de ricin ou l'huile de soja, est présent.

4. Utilisation selon la revendication 3, **caractérisée en ce que** l'agent mouillant zwitterionique est contenu dans la solution ou l'émulsion aqueuse à raison de moins de 1 % en masse, de préférence à raison de moins de 0,1 % en masse.

5. Utilisation selon la revendication 4, **caractérisée en ce que** l'agent mouillant zwitterionique est contenu dans la solution ou l'émulsion aqueuse à raison d'environ 0,05 % en masse.

6. Utilisation selon l'une des revendications 2 à 5, **caractérisée en ce que** le plastifiant non ionique est contenu dans la solution ou l'émulsion aqueuse à raison de moins de 1 % en masse, de préférence à raison de moins de 0,1 % en masse.

7. Utilisation selon la revendication 6, **caractérisée en ce que** le plastifiant non ionique est contenu dans la solution ou l'émulsion aqueuse à raison d'environ 0,075 % en masse.

8. Utilisation selon l'une des revendications 1 à 7, **caractérisée en ce que** le tensioactif cationique dans le complexe d'héparine est un composé d'ammonium ayant 4 groupes alkyle allphatiques.

9. Utilisation selon la revendication 8, **caractérisée en ce que** le composé d'ammonium est un cation Di-méthyl-di-alkyl(C10 à C18)-ammonium.

10. Utilisation selon la revendication 8 ou 9, **caractérisée en ce que** le complexe ionique est contenu dans la solution ou l'émulsion aqueuse à raison de moins de 1 % en masse, de préférence à raison d'environ 0,1 à 0,4 % en masse.

11. Utilisation selon l'une des revendications 2 à 10, **caractérisée en ce que** la solution contient un solvant organique.

12. Utilisation selon la revendication 11, **caractérisée en ce que** le solvant organique contient un alcool polaire et un hydrocarbure non polaire, de préférence dans le rapport d'environ 1:5.

13. Revêtement pour surfaces en matière plastique à activation du complément réduite constitué par un complexe ionique d'héparine anionique ou d'un dérivé d'héparine anionique et d'un tensioactif cationique où un agent mouillant zwitterionique comme dans l'une des revendications 1 à 12 est utilisé.

14. Dispositif médical ayant au moins une surface en matière plastique qui comporte un revêtement selon la revendication 13.

15. Dispositif médical selon la revendication 14, **caractérisé en ce qu'**il fait partie d'un dispositif venant en contact avec le sang, de préférence d'appareils à usage unique d'un coeur-poumon artificiel, d'un oxygénateur, d'un cathéter, d'un coeur artificiel, d'un rein artificiel, d'une membrane d'échange de gaz ou d'une prothèse vasculaire.

16. Dispositif médical selon la revendication 14 ou 15, **caractérisé en ce que** la surface en matière plastique consiste en PP, PC, PMP, PU, PE, polyester, silicone, PVC dur ou mou.
